# EUROPEAN PATENT SPECIFICATION

(11) **EP 4 706 749 B1**
(45) Date of publication and mention of the grant of the patent: **22.07.2026**
(21) Application number: 26154544.6
(22) Date of filing: 17.05.2024
(51) Int. Cl.: A61N 1/365

(54) **SYSTEM FOR CARDIAC THERAPY TO SWITCH BETWEEN DIURNAL AND NOCTURNAL PACING RATES USING CORE BODY TEMPERATURE**
SYSTEM ZUR HERZTHERAPIE ZUR UMSCHALTUNG ZWISCHEN TAGES- UND NACHTSCHRITTMACHERRATEN UNTER VERWENDUNG DER KÖRPERKERNTEMPERATUR
SYSTÈME DE THÉRAPIE CARDIAQUE POUR COMMUTER ENTRE DES FRÉQUENCES DE STIMULATION DIURNE ET NOCTURNE À L'AIDE DE LA TEMPÉRATURE CORPORELLE CENTRALE

(30) Priority: 01.06.2023 US 202363505564 P; 14.05.2024 US 202418663464
(43) Date of publication of application: 11.03.2026
(62) Divisional of application: 24176491.9
(73) Proprietor: Pacesetter, Inc., Sylmar, CA 91342 (US)
(72) Inventor: BOOTH, Daniel, West Hills, CA (US); BADIE, Nima, Sacramento, CA (US); CHIN, Donald, Palo Alto, CA (US)
(74) Representative: Barker Brettell Sweden AB

(56) References cited:
- CA-C- 1 312 923
- US-A- 4 905 697
- US-A- 5 005 574
- US-A1- 2022 072 316

## Description

### TECHNICAL FIELD

Embodiments of the present disclosure generally concern implantable medical devices, and more particularly, concern such implantable medical devices for automatically switching between diurnal and nocturnal pacing rates.

### BACKGROUND

Advances in implantable medical devices (IMD), including leadless pacemaker(s) (LP) that are implanted in the heart, have improved electrical stimulation, delays, and pacing, resulting in a better patient outcome. For most patients, the patient's heart rate will naturally be lower during sleep compared to their heart rate during the day. To better meet the physiological needs of the patient, there is a need to provide a programmable sleep or nocturnal pacing rate for IMDs to provide physiologic pacing during sleep.

Some IMDs include a sensor, such as an accelerometer, to detect motion of the patient. If no motion is detected, the IMD may determine that the patient is asleep and activate a resting pacing rate. However, the patient may not be asleep while also not being active, and it may be undesirable to lower their pacing rate to one applicable for nocturnal pacing. Also, a patient may be restless while sleeping, resulting in the IMD determining that they are awake and pacing their heart at a daytime or diurnal pacing rate.

Further, it is undesirable to make a nocturnal algorithm time-of-day dependent. For example, people travel across time zones and their circadian rhythm adapts to the new time zone, but a programmed time-of-day pacing rate change does not adapt or change to the new time zone. People work various shifts, such as during the night, evening, or early morning, that can change often or periodically, and thus the programmed pacing rates may not be reflective of the wake and sleep states of the patient. As a result, time-of-day dependent algorithms may implement both diurnal and nocturnal pacing rates at inappropriate times.

A need remains for implantable medical devices and systems that detect sleep in a patient and provide appropriate nocturnal pacing.

### SUMMARY

In accordance with embodiments herein, an implantable medical device (IMD), comprises a temperature sensor configured to sense a temperature signal indicative of a core body temperature of a patient within which the IMD is implanted, one or more pulse generators configured to generate pacing pulses, one or more electrodes configured to deliver the pacing pulses, and a control circuit. The control circuit is configured to produce a first moving composite temperature (MCT) signal based on the temperature signal sensed over a first period of time, produce a second moving composite temperature (MCT) signal based on the temperature signal over a second period of time, the second period of time being longer than the first period of time. The control circuit is configured to compare a current temperature signal to the first and second MCT signals, and control a pacing rate for the pacing pulses based on one or more relations between the current temperature signal, the first MCT signal and the second MCT signal.

Optionally, to control the pacing rate, the control circuit is configured to change the pacing rate to a nocturnal pacing rate responsive to at least one of: a) the current temperature signal falls below the second MCT signal, or b) the first MCT signal falls below the second MCT signal.

Optionally, to control the pacing rate, the control circuit is configured to change the pacing rate to a diurnal pacing rate responsive to at least one of: a) the first MCT signal rises above the second MCT signal, or b) the first MCT signal rises above a waking threshold.

Optionally, to control the pacing rate, the control circuit is configured to set the pacing rate to a diurnal pacing rate when the current temperature signal exceeds a mathematical combination of the first and second MCT signals.

Optionally, to control the pacing rate, the control circuit is configured to set one or more sleep thresholds based on i) at least one of the current temperature signal, the first MCT signal or the second MCT signal and ii) one or more offsets, the pacing rate changed responsive to at least one of the current temperature signal or the first MCT signal falling below the one or more sleep thresholds.

Optionally, to control the pacing rate, the control circuit is configured to set one or more wake thresholds based on i) at least one of the current temperature signal, the first MCT signal or the second MCT signal and ii) one or more offsets, the pacing rate changed responsive to at least one of the current temperature signal or the first MCT signal rising above the one or more wake thresholds.

Optionally, the second MCT signal represents a current extra long-term average (XLTA) temperature signal as a trend of daily average temperatures produced by summing a weighted combination of i) a prior XLTA temperature signal and ii) the current temperature signal, and the controlling the pacing rate includes setting at least one of a sleep threshold or a wake threshold based on the XLTA temperature signal and one or more offsets.

Optionally, the first period of time corresponds to one of a) to c) and the second period of time corresponds to another one of a) to c): a) a duration between 1-15 minutes, b) a duration between 1-3 hours, and c) a duration between 1-7 days.

Optionally, the control circuit includes memory configured to store program instructions and one or more processors, that when executing the program instructions, are configured to produce the first and second MCT signals by two of a) to c): a) calculating a current medium-term average (MTA) temperature signal by summing a weighted combination of i) a prior MTA temperature signal and ii) the current temperature signal, b) calculating a current long-term average (LTA) temperature signal by summing a weighted combination of i) a prior LTA temperature signal and ii) the current temperature signal, and c) calculating a current extra long-term average (XLTA) temperature signal as a trend of daily average temperatures by summing a weighted combination of i) a prior XLTA temperature signal and ii) the current temperature signal.

Optionally, the second period of time corresponds to a duration of between 1-7 days and the second MCT signal represents a multi-day nocturnal long-term average (NLTA) temperature signal that trends a daily low temperature.

Optionally, to control the pacing rate, the control circuit is configured to i) gradually decrease the pacing rate to a nocturnal pacing rate at a rate of decrease, or ii) gradually increase the pacing rate to a diurnal pacing rate at a rate of increase.

### BRIEF DESCRIPTION OF THE DRAWINGS

Figure 1A shows an external view of an implantable medical device (IMD) in accordance with embodiments herein.
Figure 1B is a schematic block diagram showing interconnection of operating elements of the IMD in accordance with embodiments herein.
Figure 2 illustrates a block diagram of a control circuit used to detect sleep and wake states in the patient and provide a temperature sensor indicated rate for pacing a heart in accordance with embodiments herein.
Figure 3 illustrates an upper graph A that shows an example of a patient's temperature and corresponding pacing rate over time, and a lower graph B that shows the corresponding moving composite temperatures determined by the control circuit over time in accordance with embodiments herein.
Figure 4 illustrates a process for dynamically adjusting the pacing rate between the diurnal pacing rate and the nocturnal pacing rate.
Figure 5 illustrates a plurality of positions in which one or more of the IMD can be implanted within a patient's heart in accordance with embodiments herein.

### DETAILED DESCRIPTION

The present invention is defined by the appended claims. Aspects, embodiments and examples described hereinafter are only of exemplary nature and are presented for better understanding the present invention.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The implantable medical devices, methods and systems described herein may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that other methods and systems may be used in accordance with an embodiment herein. Further, wherein indicated, the methods and systems may be fully or partially implemented by one or more processors of one or more devices or systems. While the operations of some methods and systems may be described as performed by the processor(s) of one device, additionally, some or all of such operations may be performed by the processor(s) of another device described herein.

### TERMS

The term "nocturnal pacing rate" shall mean a programmed pacing rate that is used by an implantable medical device (IMD) to generate pacing pulses when the patient is asleep.

The term "diurnal pacing rate" shall mean a pacing rate that is used by the IMD to generate pacing pulses when the patient is awake.

The terms "baseline pacing rate" and "programmed baseline pacing rate" shall mean a pacing rate that is used by the IMD wherein the rate is not adjusted based on factors such as exercise, activity level, etc. The nocturnal pacing rate and the diurnal pacing rate can be considered to be the baseline pacing rate when set by the IMD.

The term "diurnal" shall mean occurring or active when the patient is awake. In some cases, diurnal may correspond to conventional daytime hours, nighttime hours, or any combination thereof wherein the patient is active and/or awake.

The term "nocturnal" shall mean occurring or active when the patient is asleep. In some cases, nocturnal may correspond to conventional nighttime hours, conventional daytime hours, or any combination thereof wherein the patient is asleep.

The term "moving composite temperature (MCT) signal" shall mean a signal that is determined by one or more of data values, signals, moving averages, exponential moving averages, composites, combinations, means, medians, averages, sums, weighted combinations, normalizations, and the like. Different MCT signals can be determined using different durations of history and some MCT signals may use the same sampling rate.

The term "mathematical combination" shall mean a signal that is determined by two or more of data values, signals, moving averages, exponential moving averages, composites, combinations, means, medians, averages, sums, weighted combinations, normalizations, and the like.

The terms "raw temperature" and "raw temperature signal" shall mean a level output by a temperature sensor.

The term "current temperature signal" shall mean a raw temperature signal, a temperature signal that reflects the raw temperature signal with or without averaging, smoothing or other processing, or an MCT signal that has been determined contemporaneously or within a predetermined sampling interval.

The term "current" shall mean contemporaneously or within a predetermined sampling interval.

The term "real-time" refers to a time frame contemporaneous with episode occurrences. For example, a real-time process or operation would occur during or immediately after (e.g., within minutes or seconds after) a change in temperature or average temperature decreases below or rises above a threshold. The term "real-time," when used in connection with collecting and/or processing data utilizing an IMD, shall refer to processing operations performed substantially contemporaneous with a physiologic event of interest experienced by a patient. By way of example, in accordance with embodiments herein, temperature signals are analyzed in real time (e.g., while a patient's temperature is changing or within a few minutes after the temperature change).

The term "subcutaneous" shall mean below the skin, but not intravenous. For example, a subcutaneous electrode/lead does not include an electrode/lead located in a chamber of the heart, in a vein on the heart, or in the lateral or posterior branches of the coronary sinus.

The terms "processor," "a processor", "one or more processors" and "the processor" shall mean one or more processors. The one or more processors may be implemented by one, or by a combination of more than one implantable medical device, a wearable device, a local device, a remote device, a server computing device, a network of server computing devices and the like. The one or more processors may be implemented at a common location or at distributed locations. The one or more processors may implement the various operations described herein in a serial or parallel manner, in a shared-resource configuration and the like.

The terms "cardiac signals", "cardiac activity", "CA signal" and "CA signals" (collectively "CA signals") are used interchangeably throughout and shall mean measured signals indicative of cardiac activity by a region or chamber of interest. For example, the CA signals may be indicative of impedance, electrical or mechanical activity by one or more chambers (e.g., left or right ventricle, left or right atrium) of the heart and/or by a local region within the heart (e.g., impedance, electrical or mechanical activity at the AV node, along the septal wall, within the left or right bundle branch, within the purkinje fibers). The cardiac activity may be normal/healthy or abnormal/arrhythmic. An example of CA signals includes EGM signals. Electrical based CA signals refer to an analog or digital electrical signal recorded by two or more electrodes, where the electrical signals are indicative of cardiac activity. Heart sound (HS) based CA signals refer to signals output by a heart sound sensor such as an accelerometer, where the HS based CA signals are indicative of one or more of the S1, S2, S3 and/or S4 heart sounds. Impedance based CA signals refer to impedance measurements recorded along an impedance vector between two or more electrodes, where the impedance measurements are indicative of cardiac activity

The term "health care system" shall mean a system that includes equipment for measuring health parameters, and communication pathways from the equipment to secondary devices. The secondary devices may be at the same location as the equipment, or remote from the equipment at a different location. The communication pathways may be wired, wireless, over the air, cellular, in the cloud, etc. In one example, the healthcare system provided may be one of the systems described in U.S. Patent Application No. 2021/0020294 entitled METHODS DEVICE AND SYSTEMS FOR HOLISTIC INTEGRATED HEALTHCARE PATIENT MANAGEMENT. Other patents that describe example monitoring systems include U.S. Pat. No. 6,572,557; entitled SYSTEM AND METHOD FOR MONITORING PROGRESSION OF CARDIAC DISEASE STATE USING PHYSIOLOGIC SENSORS; U.S. Pat. No. 6,480,733 entitled METHOD FOR MONITORING HEART FAILURE; U.S. Pat. No. 7,272,443 entitled SYSTEM AND METHOD FOR PREDICTING A HEART CONDITION BASED ON IMPEDANCE VALUES USING AN IMPLANTABLE MEDICAL DEVICE; U.S. Pat. No. 7,308,309 entitled DIAGNOSING CARDIAC HEALTH UTILIZING PARAMETER TREND ANALYSIS; and U.S. Pat. No. 6,645,153 entitled SYSTEM AND METHOD FOR EVALUATING RISK OF MORTALITY DUE TO CONGESTIVE HEART FAILURE USING PHYSIOLOGIC SENSORS.

The term "IMD" shall mean an implantable medical device. Embodiments may be implemented in connection with one or more implantable medical devices (IMDs). Non-limiting examples of IMDs include one or more of neurostimulator devices, implantable leadless monitoring and/or therapy devices, and/or alternative implantable medical devices. For example, the IMD may represent a subcutaneous cardioverter defibrillator, cardiac monitoring device, pacemaker, cardioverter, cardiac rhythm management device, defibrillator, neurostimulator, leadless monitoring device, leadless pacemaker, left ventricular assist device (LVAD), and the like. The IMD may measure electrical and/or mechanical information. For example, the IMD may include one or more structural and/or functional aspects of the device(s) described in U.S. Patent Number 9,333,351, entitled "NEUROSTIMULATION METHOD AND SYSTEM TO TREAT APNEA" and U.S. Patent Number 9,044,610, entitled "SYSTEM AND METHODS FOR PROVIDING A DISTRIBUTED VIRTUAL STIMULATION CATHODE FOR USE WITH AN IMPLANTABLE NEUROSTIMULATION SYSTEM". The IMD may monitor transthoracic impedance, such as implemented by the CorVue algorithm offered by St. Jude Medical. Additionally or alternatively, the IMD may include one or more structural and/or functional aspects of the device(s) described in U.S. Patent Number 9,216,285, entitled "LEADLESS IMPLANTABLE MEDICAL DEVICE HAVING REMOVABLE AND FIXED COMPONENTS" and U.S. Patent Number 8,831,747, entitled "LEADLESS NEUROSTIMULATION DEVICE AND METHOD INCLUDING THE SAME". Additionally or alternatively, the IMD may include one or more structural and/or functional aspects of the device(s) described in U.S. Patent Number 8,391,980, entitled "METHOD AND SYSTEM FOR IDENTIFYING A POTENTIAL LEAD FAILURE IN AN IMPLANTABLE MEDICAL DEVICE" and U.S. Patent Number 9,232,485, entitled "SYSTEM AND METHOD FOR SELECTIVELY COMMUNICATING WITH AN IMPLANTABLE MEDICAL DEVICE". Additionally or alternatively, the IMD may be a subcutaneous IMD that includes one or more structural and/or functional aspects of the device(s) described in U.S. Patent Number 10,765,860, entitled "SUBCUTANEOUS IMPLANTATION MEDICAL DEVICE WITH MULTIPLE PARASTERNAL-ANTERIOR ELECTRODES"; U.S. Patent Number 10,722,704, entitled "IMPLANTABLE MEDICAL SYSTEMS AND METHODS INCLUDING PULSE GENERATORS AND LEADS"; U.S. Patent Number 11,045,643, entitled "SINGLE SITE IMPLANTATION METHODS FOR MEDICAL DEVICES HAVING MULTIPLE LEADS". Further, one or more combinations of IMDs may be utilized from the above patents and applications in accordance with embodiments herein. Embodiments may be implemented in connection with one or more subcutaneous implantable medical devices (S-IMDs). For example, the S-IMD may include one or more structural and/or functional aspects of the device(s) described in U.S. Patent Number 10,722,704, entitled "IMPLANTABLE MEDICAL SYSTEMS AND METHODS INCLUDING PULSE GENERATORS AND LEADS"; U.S. Patent Number 10,765,860, entitled "SUBCUTANEOUS IMPLANTATION MEDICAL DEVICE WITH MULTIPLE PARASTERNAL-ANTERIOR ELECTRODES". The IMD may represent a passive device that utilizes an external power source, and entirely mechanical plan will device, and/or an active device that includes an internal power source. The IMD may deliver some type of therapy/treatment, provide mechanical circulatory support, and/or merely monitor one or more physiologic characteristics of interest (e.g., PAP, CA signals, impedance, heart sounds).

Additionally or alternatively, embodiments herein may be implemented in connection with an integrated healthcare patient management system or network, such as described in U.S. Patent Application Number 2021/0020294, entitled "METHODS, DEVICE AND SYSTEMS FOR HOLISTIC INTEGRATED HEALTHCARE PATIENT MANAGEMENT".

Additionally or alternatively, embodiments herein may be implemented in connection with the methods and systems described in U.S. Patent Application Number 2021/0350931, entitled "METHOD AND SYSTEM FOR HEART CONDITION DETECTION USING AN ACCELEROMETER".

Additionally or alternatively, embodiments herein may be implemented in connection with the methods and systems described in U.S. Patent Application 2021/0361945, entitled "System and method for rate modulated cardiac therapy utilizing a temperature sensor"; U.S. Patent Number 5,005,574, entitled "Temperature-based, rate-modulated cardiac therapy apparatus and method"; U.S. Patent Number 4,905,697, entitled "Temperature-controlled cardiac pacemaker responsive to body motion"; and U.S. Patent Number 4,945,909, entitled "Pacemaker with activity-dependent rate limiting".

### System Overview

In accordance with new and unique aspects herein, systems and methods are described that automatically detect sleep, and waking from sleep, in a patient and provide appropriate nocturnal pacing and diurnal pacing, respectively. Core body temperature and intrinsic heart rate have circadian rhythms which correlate to metabolic demand and gradually lower before sleep and continue to lower during sleep until they reach nightly minimums. Before a person wakes up and while they are waking, the temperature and intrinsic heart rate rise to daytime baselines. As discussed further herein, a sleep detection algorithm identifies when to lower the pacing rate (e.g., diurnal pacing rate) of a pacemaker to a programmed nocturnal pacing rate that is slower or below the programmed diurnal pacing rate, by comparing signals related to the patient's temperature. For example, a first signal, such as a first moving composite temperature (MCT) signal that is determined over a first length of time can be compared with a second MCT signal that is determined over a second length of time that is longer than the first length of time. When the first signal falls below (e.g., drops below) the second MCT signal, the pacing rate is gradually lowered toward the nocturnal pacing rate. Also, a wake detection algorithm uses the patient's temperature signal to identify when to raise the nocturnal pacing rate to the diurnal pacing rate. For example, the first signal is compared to the second or other identified MCT signal(s) and/or threshold(s). When the first MCT signal exceeds (e.g., rises above) the second or other MCT signal(s) and/or threshold(s), the IMD gradually raises the pacing rate back to the diurnal pacing rate.

A technical effect of the various embodiments herein is to determine, based on a comparison of the patient's current temperature signal, one or more MCT signals, and/or one or more threshold, whether the IMD should trigger the sleep detection algorithm or the wake detection algorithm. A technical effect of triggering the sleep detection algorithm is automatically implementing a decrease in the pacing rate, at a predetermined programmable rate, to a nocturnal pacing rate. A technical effect of triggering the wake detection algorithm is automatically implementing an increase in the pacing rate, at a predetermined programmable rate, to a diurnal pacing rate.

Advantageously, the IMD measures changes in core body temperature of the patient and does not use time-of-day information to determine whether the patient is awake or asleep. In some embodiments, the core body temperature measurements come from a leadless pacemaker (LP) implanted intracardially. The measurement could be made by any single or multiple LP(s), where multiple LPs could communicate, coordinate, and apply the same system-wide pacing rate. In other embodiments, the core body temperature measurements can be acquired by a different sensor or other implantable device that is capable of pacing the patient and/or transmits the temperature data to the LP or other pacing IMD.

Further advantages of embodiments herein include determining whether the patient is awake or asleep without relying on patient movement detected by an IMD. Patient movement can occur during sleep and a patient can be very still when awake, and thus confusion experienced by other algorithms attempting to set diurnal and nocturnal pacing rates based on movement, posture, and the like are avoided.

Although the embodiments herein are described primarily in relation to one or more LPs, it should be understood that the embodiments herein may be implemented using one or more other types of implantable medical devices, such as but not limited to, a subcutaneous cardioverter defibrillator, cardiac monitoring device, pacemaker, cardioverter, cardiac rhythm management device, defibrillator, neurostimulator, leadless monitoring device, left ventricular assist device, and the like, that include a temperature sensor for sensing a patient's core temperature, or are configured to receive signals from a temperature sensor. For example, one or more LPs may be used, a single implantable device, and/or two or more implantable devices. Alternatively or additionally, an IMD capable of pacing may receive core body temperature and/or diurnal and nocturnal pacing information from another implantable device.

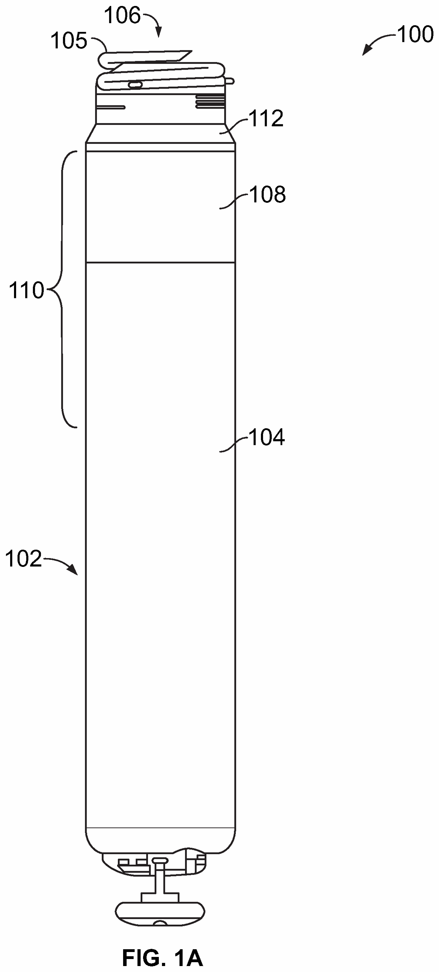

Figure 1A shows an external view of an implantable medical device (IMD) 100 in accordance with embodiments herein. The IMD 100 in the embodiment shown is a leadless pacemaker (LP); however, the systems and methods disclosed herein are not limited to use within an LP. The IMD 100 can include a hermetic housing 102 with electrodes 104 and 106 located within, on, or near the housing 102, for delivering pacing pulses to and sensing electrical activity from the muscle of the cardiac chamber, and for bidirectional communication with at least one other device within or outside the body (e.g., RF, conductive communication). In various embodiments, the electrodes 104 and 106 can be coupled on, within, or within a distance, such as approximately two centimeters of the housing 102. In alternative embodiments, the electrodes 104 and 106 can be coupled on, within, or within approximately fifteen centimeters of the housing 102. In some arrangements, the electrodes 104 and 106 can be formed integrally to an outer surface of the housing 102.

As shown, electrode 106 can be separated from but surrounded partially by a fixation mechanism 105, and the electrode 104 can be disposed on the housing 102. The fixation mechanism 105 can be a fixation helix, a plurality of hooks, barbs, or other attaching features configured to attach the pacemaker to tissue, such as heart tissue.

The housing 102 can also include an electronics compartment 110 within the housing 102 that contains the electronic components necessary for operation of the pacemaker, including, for example, a pulse generator, communication electronics, a battery, and a control circuit. The control circuit may include one or more processor, hardware, firmware, etc., and combinations thereof. The hermetic housing 102 can be adapted to be implanted on or in a human heart, and can be cylindrically shaped, rectangular, spherical, or any other appropriate shapes, for example.

The housing 102 can comprise a conductive, biocompatible, inert, and anodically safe material such as titanium, 316L stainless steel, or other similar materials. The housing 102 can further comprise an insulator disposed on the conductive material to separate electrodes 104 and 106. The insulator can be an insulative coating on a portion of the housing between the electrodes 104, 106, and can comprise materials such as silicone, polyurethane, parylene, or another biocompatible electrical insulator commonly used for implantable medical devices. As shown in Figure 1A, a single insulator 108 is disposed along the portion of the housing 102 between electrodes 104 and 106. In some embodiments, the housing 102 itself can comprise an insulator instead of a conductor, such as an alumina ceramic or other similar materials, and the electrodes 104, 106 can be disposed upon the housing.

The IMD 100 can further include a header assembly 112 to isolate electrode 104 from electrode 106. The header assembly 112 can be made from PEEK, tecothane or another biocompatible plastic, and can contain a ceramic to metal feedthrough, a glass to metal feedthrough, or other appropriate feedthrough insulator as known in the art.

The electrodes 104 and 106 can comprise pace/sense electrodes, and/or return electrodes. A low-polarization coating can be applied to the electrodes, 104, 106 such as sintered platinum, platinum-iridium, iridium, iridium-oxide, titanium-nitride, carbon, or other materials commonly used to reduce polarization effects, for example. In some cases, the electrode 106 can be a pace/sense electrode and the electrode 104 can be a return electrode. The electrode 104 can be a portion of the conductive housing 102 that does not include an insulator 108.

Several techniques and structures can be used for attaching the housing 102 to the interior or exterior wall of the heart. A helical fixation mechanism 105 can enable insertion of the device endocardially or epicardially through a guiding catheter. A torqueable catheter can be used to rotate the housing 102 and force the fixation mechanism 105 into heart tissue, thus affixing the fixation mechanism 105 (and also the electrode 106 in Figure 1A) into contact with stimulable tissue. Electrode 104 can serve as an indifferent electrode for sensing and pacing. The fixation mechanism 105 may be coated partially or in full for electrical insulation, and a steroid-eluting matrix may be included on or near the device to minimize fibrotic reaction, as is known in conventional pacing electrode-leads.

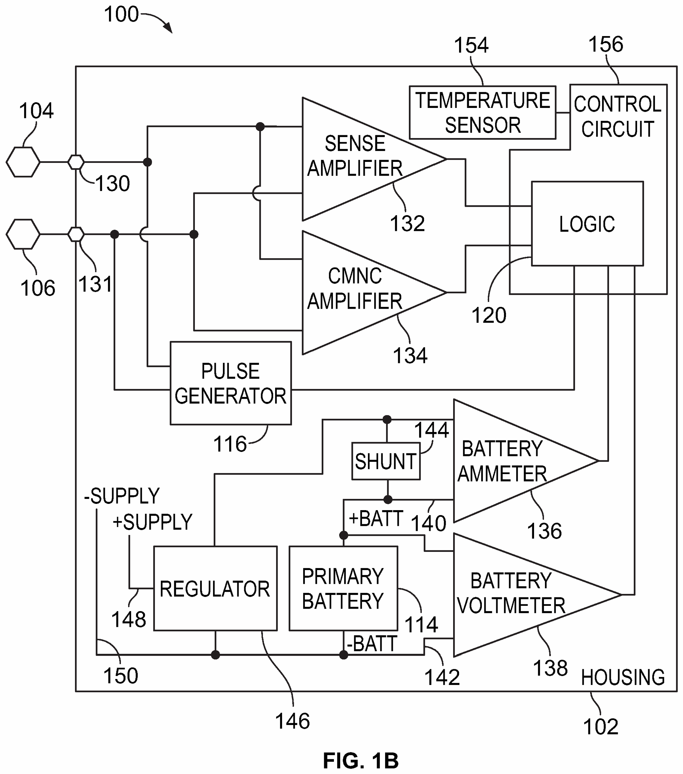

Turning to Figure 1B, a schematic block diagram depicts an embodiment of the IMD 100 in accordance with embodiments herein. The IMD 100 comprises the housing 102, electrodes 104 and 106 coupled to the housing 102, a pulse delivery system hermetically contained within the housing 102 and electrically coupled to the electrodes 104 and 106. The pulse delivery system is configured for sourcing energy internal to the housing 102 and generating and delivering electrical pulses to the electrodes 104 and 106. The IMD 100 further comprises a temperature sensor 154 which may be enclosed within the housing 102 or may be supported by the housing 102 and adapted to sense temperature. A control circuit 156 is hermetically contained within the housing 102 and communicatively coupled to the pulse generator 116, the temperature sensor 154 and the electrodes 104 and 106. The control circuit 156 is configured to perform the functionality described herein. The control circuit 156 may include logic 120, for example one or more processors, controller, central processing unit, state machine, programmable logic array, and the like. Logic 120 may control the pulse generator 116 to deliver electrical pulses with the amplitudes, pulse widths, frequency, or electrode polarities specified by selected one or more therapy programs stored in a memory. The temperature sensor 154 senses a temperature signal indicative of a core body temperature of the patient within which the IMD 100 is implanted. Temperature sensors 154 are known in the art and thus are not limited to any particular implementation of sensor. The control circuit 156 is used to provide a temperature sensor indicated rate for pacing a heart according to certain embodiments of the disclosure. The temperature sensor indicated rate from the control circuit 156 is used by the logic 120 to generate control signals specifying stimulation therapy, such as pacing rate, sent to the pulse generator 116.

The control circuit 156 and other blocks can be implemented by software, firmware, or combinations thereof. A software module may reside in RAM memory, flash memory, ROM memory, EPROM memory, EEPROM memory, registers, or any other form of storage medium known in the art. An exemplary storage medium is coupled to the processor such that the processor can read information from, and write information to, the storage medium. In the alternative, the storage medium may be integral to the processor. In certain embodiments, the logic 120 comprises an application-specific integrated circuit (ASIC) and the temperature sensor 154 comprises a semiconductor temperature sensor incorporated into the ASIC. The control circuit 156 and storage medium may reside in the ASIC. In certain embodiments, logic 120 can comprise a single ultra-low power ASIC chip configured to sense, pace, and communicate. The logic 120 can control electrical pulse delivery.

The control circuit 156 can be programmable by communication signals transmitted via the electrodes 104 and 106. The information communicated on the incoming communication channel can include, but is not limited to pacing rate, pulse duration, sensing threshold, and other parameters commonly programmed externally in typical pacemakers, as well as a temperature signal generated by an external temperature sensor (not shown). The information communicated on the outgoing communication channel can include, but is not limited to programmable parameter settings, event counts (pacing and sensing), battery voltage, battery current, and other information commonly displayed by external programmers used with pacemakers, as well as a temperature signal. The outgoing communication channel can also echo information from the incoming channel, to confirm correct programming.

Hermetic feedthroughs 130, 131 conduct electrode signals through the housing 102. The housing 102 contains a primary battery 114 to provide power for pacing, sensing, and communication. The housing 102 contains circuits 132 for sensing cardiac signals / cardiac activity from the electrodes 104 and 106; circuits 134 for receiving information from at least one other device via the electrodes 104 and 106; and a pulse generator 116 for generating pacing pulses for delivery via the electrodes 104 and 106 and also for transmitting information to at least one other device via the electrodes 104 and 106. The IMD 100 further contains circuits for monitoring device health, for example a battery current monitor 136 and a battery voltage monitor 138. Logic 120 controls these operations in a predetermined manner.

The primary battery 114 has positive terminal 140 and negative terminal 142. In certain embodiments, the battery is a lithium carbon monofluoride (Li/CFx) battery. Current from the positive terminal 140 of primary battery 114 flows through a shunt 144 to a regulator circuit 146 to create a positive voltage supply 148 suitable for powering the remaining circuitry of the IMD 100. The shunt 144 enables the battery current monitor 136 to provide the logic 120 with an indication of battery current drain and indirectly of device health.

In various embodiments, the system can manage power consumption to draw limited power from the battery 114, thereby reducing device volume. Each circuit in the system can be designed to avoid large peak currents. For example, cardiac pacing can be achieved by discharging a tank capacitor (not shown) across the pacing electrodes. Recharging of the tank capacitor is typically controlled by a charge pump circuit. In a particular embodiment, the charge pump circuit is throttled to recharge the tank capacitor at constant power from the battery 114.

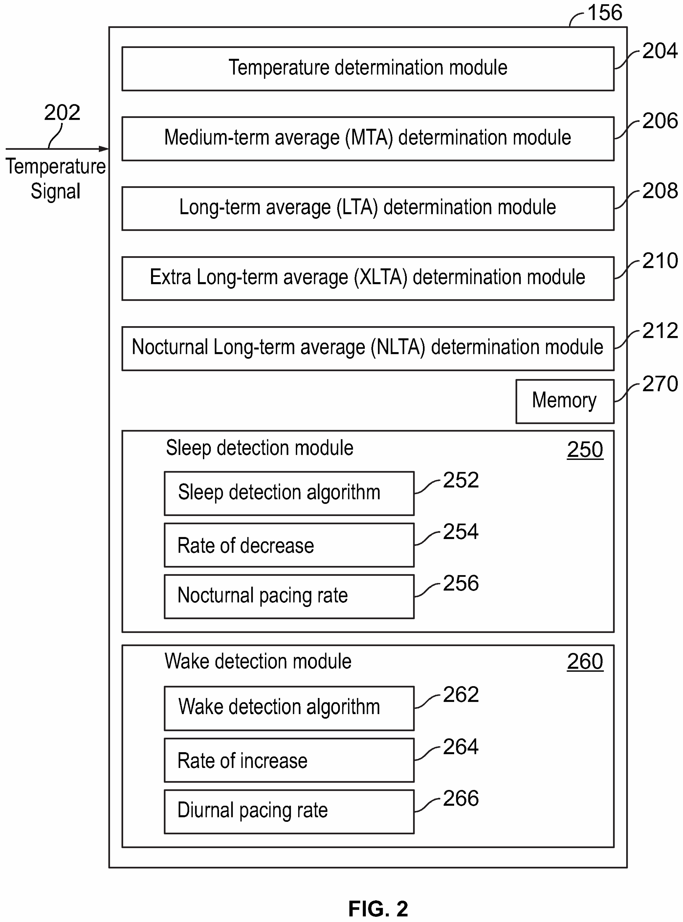

Figure 2 illustrates a block diagram of the control circuit 156 used to detect sleep and wake states in the patient and provide a temperature sensor indicated rate for pacing a heart in accordance with embodiments herein. Although not shown, the control circuit 156 can include components such as e.g., filters, summers, integrators, etc., that can be implemented in the digital domain using digital circuitry, software, and/or firmware. However, it is noted that in alternative embodiments of the present technology the control circuit 156 can instead be implemented using analog components. In the embodiments described herein it is assumed that the blood temperature measured by a temperature sensor 154 is the same or substantially the same as the core body temperature. Accordingly, the terms blood temperature and core body temperature may be used interchangeably.

A raw temperature signal 202 is provided to the control circuit 156 from the temperature sensor 154 (or other temperature sensor if being used, including temperature sensors located outside of the IMD 100 and/or within other implantable devices). The control circuit 156 controls the interval in which the raw temperature signal 202 detected by the temperature sensor 154 is sampled by the control circuit 156 and/or modules within the control circuit 156. In this illustrative embodiment, the temperature signal 202 is sampled at a sampling interval of five seconds. However, sampling intervals that are greater than or less than five seconds are also possible and within the scope of the embodiments described herein.

A temperature determination module 204 can determine a temperature signal based on the raw temperature signal 202. The temperature signal can be determined at regular sampling intervals (e.g., every 5 seconds) and may or may not employ smoothing over a short period of time.

The control circuit 156 also can calculate a plurality of moving composite temperature (MCT) signals over different periods of time. In some embodiments, the MCT signals can be determined at regular sampling intervals (e.g., every 5 seconds) and can be one or more of moving averages, exponential moving averages, composites, combinations, means, averages, sums, weighted combinations, etc., based on previously computed signals, the raw temperature signal 202, and the like. The raw temperature signal 202, the temperature signal based on the raw temperature signal 202, and/or one or more MCT signals can be used to identify sleep and wake times.

Although the calculation of the MCT signals is discussed in terms of moving averages, it should be understood that the MCT signals can be determined using other mathematical algorithms and/or variations, and are not limited to those discussed herein. Further, time constants can be smaller or greater than in the examples provided.

A medium-term average (MTA) determination module 206 determines an MTA temperature signal that has a relatively smaller time constant, and thus will generally follow the temperature of the raw temperature signal 202. In some embodiments, the MTA temperature signal can be computed by summing two weighted terms where the weight is based on a time constant (e.g., expressed as the number of samples taken within approximately 1-15 minutes). The two weighted terms can be i) previously calculated MTA (e.g., prior MTA) multiplied by a weight (1 - [1 / time constant]), and ii) raw temperature divided by the time constant. By way of example only, if the time constant is 10 minutes and the sampling rate is every 5 seconds, 128 samples will be taken. In other embodiments, a short-term average (STA) can be used to determine the MTA, wherein the MTA has a minimum function applied to it: MTA = min(MTA, STA). The STA temperature signal can be computed in the same manner as a long term average (LTA) (discussed below), but with a very short time constant, such as approximately 20 seconds. This enables the MTA to lower quickly (e.g., as fast or nearly as fast as the STA) as sleep approaches.

A long-term average (LTA) determination module 208 determines an LTA temperature signal that has a relatively larger time constant than the MTA temperature signal. In some embodiments, the LTA temperature signal can be computed by summing two weighted terms where the weight is based on a time constant (e.g., expressed as the number of samples taken within approximately 85 minutes). The two weighted terms can be i) previously calculated LTA (e.g., prior LTA) multiplied by a weight (1 - [1 / time constant]), and ii) raw temperature divided by the time constant.

An extra long-term (multi-day) average (XLTA) determination module 210 determines an XLTA temperature signal that has a relatively larger time constant than the LTA temperature signal. The XLTA temperature signal trends the daily average temperature output by the raw temperature signal 202. In some embodiments, the XLTA temperature signal can be computed by summing two weighted terms where the weight is based on a time constant (e.g., expressed as the number of samples taken within approximately 1-7 days). The two weighted terms can be i) previously calculated XLTA (e.g., prior XLTA) multiplied by a weight (1 - [1 / time constant]), and ii) raw temperature divided by the time constant.

A multi-day nocturnal long-term average (NLTA) determination module 212 determines an NLTA temperature signal that trends the daily low temperature. The NLTA determination module 212 computes the NLTA temperature signal differently depending on temperature fall or rise, by summing two weighted terms, where the weight is based on two different time constants. Each time constant is expressed as the number of samples taken within approximately 1-15 minutes (for temperature fall) or approximately 1-7 days (for temperature rise or temperature constant). The two weighted terms can be i) previously calculated NLTA (e.g., prior NLTA) multiplied by a weight (1 - [1 / specific time constant]), and ii) raw temperature divided by the specific time constant.

The time constants and weights expressed herein are examples and thus the embodiments are not limited to the specific numbers and/or ranges. Further, to the extent that the terms MTA, LTA, XLTA, and NLTA, etc., are used, it should be understood that the time ranges, moving averages, etc., are relative to each other, and are not specific to a period of time or a specific length of time.

For example, a first MCT signal is produced, such as by the control circuit 156, based on the temperature signal 202 sensed over a first period of time and a second MCT signal is produced based on the temperature signal 202 sensed over a second period of time that is longer than the first period of time. In some embodiments, the first period of time corresponds to a duration between 1-15 minutes, 1-3 hours, or 1-7 days, and the second period of time corresponds to another one of 1-15 minutes, 1-3 hours, or 1-7 days. In further embodiments, the first and second MCT signals are produced by two of a) calculating a current MTA temperature signal by summing a weighted combination of i) prior MTA temperature signal and ii) the current temperature signal; b) calculating a current LTA temperature signal by summing a weighted combination of i) a prior LTA temperature signal and ii) the current temperature signal; and c) calculating a current XLTA temperature signal as a trend of daily average temperatures by summing a weighted combination of i) a prior XLTA temperature signal and ii) the current temperature signal. In still further embodiments, the second period of time corresponds to a duration of between 1-10 days and the second MCT signal represents a multi-day NLTA temperature signal that trends a daily low temperature.

Outputs from the temperature determination module 204, MTA determination module 206, LTA determination module 208, XLTA determination module 210, and NLTA determination module 212 are received by a sleep detection module 250 and a wake detection module 260. Although the sleep and wake detection modules 250, 260 are shown separately for discussion, it should be understood that processing to determine sleep and wake can be integrated together, as discussed further herein. The sleep detection module 250 and the wake detection module 260 control a pacing rate of pacing pulses based on whether the control circuit 156 determines that the patient is asleep or awake.

Referring first to the sleep detection module 250, the nocturnal pacing can be triggered when a sleep detection algorithm 252 determines that one of the signals (raw temperature signal, current temperature signal, MTA temperature signal, etc.) has crossed one or more predetermined threshold(s). For example, the sleep detection algorithm 252 can determine that the signal has fallen or dropped below one or more other signals (LTA temperature signal, XLTA temperature signal, a predetermined sleep threshold, a sleep threshold determined through a mathematical combination of two or more of the temperature, MTA, XLTA, NLTA temperature signals, threshold(s) based on population data, threshold(s) determined specifically for the patient, etc.). When the sleep detection algorithm 252 determines that the IMD 100 should be paced at the programmable nocturnal pacing rate 256, the control circuit 156 directs the IMD 100 to gradually lower the pacing rate at a programmable rate of decrease 254 to the nocturnal pacing rate 256.

Referring to the wake detection module 260, the diurnal pacing can be triggered when a wake detection algorithm 262 determines that one of the signals (raw temperature signal, current temperature signal, MTA temperature signal, etc.) has crossed one or more predetermined threshold(s). For example, the sleep detection algorithm 252 can determine that the signal has risen above one or more other signals (LTA temperature signal, XLTA temperature signal, a predetermined wake threshold, a wake threshold determined through a mathematical combination of two or more of the temperature, MTA, XLTA, NLTA temperature signals, threshold(s) based on population data, threshold(s) determined specifically for the patient, etc.). When the wake detection algorithm 262 determines that the IMD 100 should be paced at the programmable diurnal pacing rate 266, the control circuit 156 directs the IMD 100 to gradually increase the pacing rate at a programmable rate of increase 264 to the diurnal pacing rate 266.

Memory 270 can be included within the control circuit 156, the logic 120, and/or elsewhere within the IMD 100 and be communicatively coupled with the control circuit 156. The memory 270 can store program instructions that can be executed by one or more processors, such as within the control circuit 156, to generate the temperature signal based on the temperature signal 202, determine the MTA, LTA, XLTA, and NLTA signals, and control the pacing rates by determining whether the nocturnal pacing rate 256 or the diurnal pacing rate 266 should be active and used as the IMD's baseline pacing rate. The rate of decrease 254, nocturnal pacing rate 256, rate of increase 264, and diurnal pacing rate 266 can also be stored in the memory 270.

In some embodiments, one or more processors, such as of the sleep detection module 250 and/or wake detection module 260, compare a current temperature signal to the first and second MCT signals. In other embodiments, the one or more processors compare more than two MCT signals to determine a relationship between the MCT signals. The one or more processors, such as of the control circuit 156, control a pacing rate for pacing pulses that are generated by one or more pulse generators 116 and delivered to the one or more electrodes 104, 106, based on one or more relations between the current temperature signal, the first MCT signal and the second MCT signal.

In some embodiments, controlling the pacing rate includes changing the pacing rate to a nocturnal pacing rate 256 responsive to at least one of a) the current temperature signal falls below the second MCT signal, or b) the first MCT signal falls below the second MCT signal. In other embodiments, controlling the pacing rate includes changing the pacing rate to the diurnal pacing rate 266 responsive to at least one of a) the first MCT signal rises above the second MCT signal, or b) the first MCT signal rises above a waking threshold. In still further embodiments, controlling the pacing rate includes setting the pacing rate at a diurnal pacing rate 266 when the current temperature signal exceeds a mathematical combination of the first and second MCT signals.

In some embodiments, an offset could be applied to the one or more thresholds, such that the pacing rate is decreased or increased sooner or later. For example, the sleep detection module 250 can be programmed to initiate the rate of decrease 254 sooner or later, and the wake detection module 260 can be programmed to initiate the rate of increase 264 sooner or later, than would be accomplished without the offset. It should be understood that different offsets can be used by the sleep and wake detection algorithms 252, 262.

In some embodiments, controlling the pacing rate includes setting one or more sleep thresholds based on i) at least one of the current temperature signal, the first MCT signal, or the second MCT signal, and ii) one or more offsets, the pacing rate changed responsive to at least one of the current temperature signal or the first MCT signal falling below the one or more sleep thresholds. In other embodiments, controlling the pacing rate includes setting one or more wake thresholds based on i) at least one of the current temperature signal, the first MCT signal, or the second MCT signal, and ii) one or more offsets, the pacing rate changed responsive to at least one of the current temperature signal or the first MCT signal rising above the one or more wake threshold. In yet further embodiments, the second MCT signal represents a current extra XLTA temperature signal as a trend of daily average temperatures produced by summing a weighted combination of i) a prior XLTA temperature signal and ii) the current temperature signal, the controlling the pacing rate includes setting at least one of a sleep threshold or a wake threshold based on the XLTA temperature signal and one or more offsets.

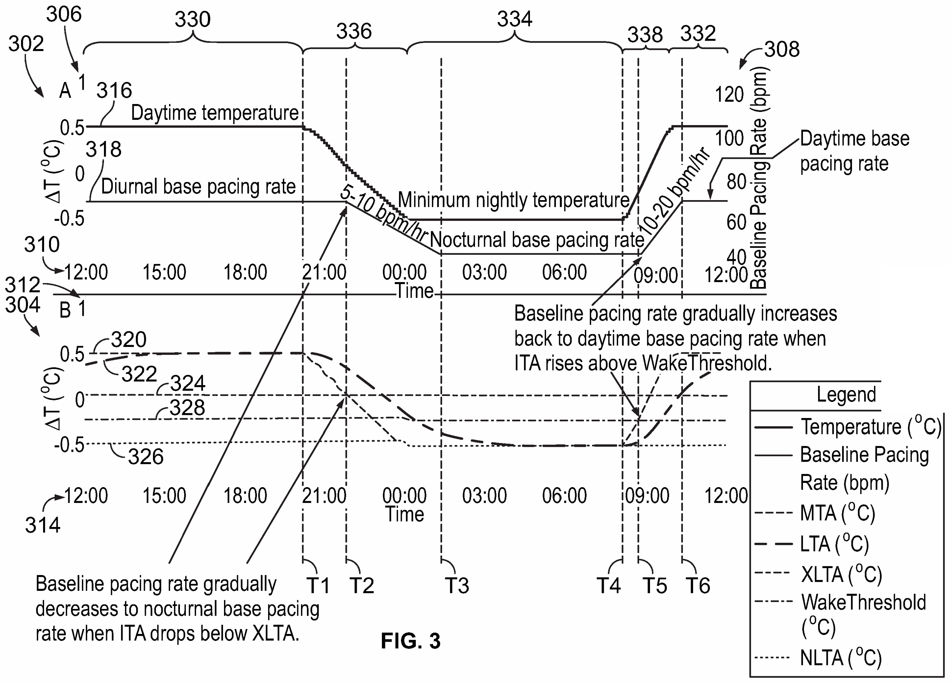

Figure 3 illustrates an upper graph A 302 that shows an example of a patient's temperature and corresponding pacing rate over time, and a lower graph B 304 that shows the corresponding moving composite temperatures determined by the control circuit 156 over time in accordance with embodiments herein. Left vertical axis 306 of the upper graph A 302 plots change in temperature in Celsius (other units such as Fahrenheit may be used), right vertical axis 308 plots the baseline pacing rate (e.g., programmed pacing rate unchanged by other factors or algorithms) in beats-per-minute (bpm), and horizontal axis 310 plots time (e.g., 24 hour clock) from noon (12:00 pm) on the left side to noon (12:00) pm on the right side. Left vertical axis 312 of the lower graph B 304 plots change in temperature and horizontal axis 314 plots time. It is recognized that the temperature changes, pacing rates and time illustrated in Figure 3 are examples and are not limiting. The upper graph A 302 and lower graph B 304 will be discussed together with Figure 2.

The upper graph A 302 illustrates an exemplary patient's temperature signal 316 that can be determined using the temperature sensor 154 and the control circuit 156 of the IMD 100. The temperature signal 316 can be indicative of the raw temperature or core body temperature, and in some embodiments can be indicative of a non-processed temperature signal, while in other embodiments can have smoothing, averaging, etc., applied over a period of time that is relatively shorter than the period of time used to determine the MTA temperature signal. Daytime temperature is indicated within times 330 and 332, minimum nightly temperature is indicated within time 334, the decrease in temperature from daytime to nighttime within time 336 and increase in temperature from nighttime to daytime within time 338. The upper graph A 302 also illustrates the patient's associated pacing rate 318.

The lower graph B 304 illustrates the MTA temperature signal 320, LTA temperature signal 322, XLTA temperature signal 324, and NLTA temperature signal 326 that can be determined by one or more processors of the control circuit 156. The lower graph B 304 also illustrates a wake threshold 328 that is illustrated as approximately halfway between the NLTA temperature signal 326 and the XLTA temperature signal 324 (e.g., mean(XLTA, NLTA), a mathematical combination of two MCT signals). In other embodiments, the wake threshold 328 is programmable and can be set to be closer to the NLTA temperature signal 326 or the XLTA temperature signal 324 such as by a healthcare practitioner.

As daytime temperature stabilizes, as shown within the times 330 and 332, the temperature signal 316 is relatively constant. Correspondingly, within the times 330 and 332, the MTA temperature signal 320 and LTA temperature signal 322 become approximately equal values. The NLTA temperature signal 326 typically remains near the minimum nightly temperature, as indicated within the time 334. The XLTA temperature signal 324 typically remains approximately halfway between the NLTA temperature signal 326 and the average day temperature.

By circadian rhythm, the patient's temperature, and thus the temperature signal 316, gradually decreases as nocturnal sleep approaches, as shown at T1 in time 336. Following T1, the MTA temperature signal 320 also decreases, falling below the LTA temperature signal 322 which triggers the control circuit 156 to use the XLTA temperature signal 324 as a sleep threshold for detecting sleep.

When the MTA temperature signal 320 falls below the XLTA temperature signal 324 at T2, the control circuit 156 gradually decreases the pacing rate toward a programmable nocturnal pacing rate 256 that is set at T3. The rate at which the pacing rate gradually decreases from the diurnal pacing rate 266 to the nocturnal pacing rate 256 is, in some embodiments, approximately 5-10 beats per minute per hour (bpm/hr), as programmably defined in the rate of decrease 254. The rate of decrease 254 can be greater than or less than approximately 5-10 bpm per hour and is configurable, such as by a healthcare practitioner using an external device (e.g., programmer). In some embodiments, pacing rate is decreased gradually, over time, such as to follow heart rate trends typical of healthy patients. The rate of decrease 254 can avoid sudden, non-linear jumps in pacing rate. Therefore, the rate of decrease 254 can be accomplished by spreading the bpm/hr decrease over time. For example, if the rate of decrease 254 is 6 bpm/hr, the pacing rate is decreased by 1 bpm every 10 minutes until the nocturnal pacing rate 256 is accomplished.

By circadian rhythm, the patient's temperature and thus the temperature signal 316 begins to gradually increase, such as at T4, as the patient approaches an awakened state. Following closely, the MTA temperature signal 320 also increases and rises above the LTA temperature signal 322, which triggers the control circuit 156 to use the wake threshold 328 as a threshold for initiating the increase in the pacing rate to the diurnal pacing rate 266.

When the MTA temperature signal 320 rises above the wake threshold 328 at T5, the pacing rate gradually increases, such as at a configurable rate of approximately 10-20 bpm/hr (e.g., rate of increase 264) toward the diurnal pacing rate 266, which is reached at T6. The rate of increase 264 can be greater than or less than approximately 10-20 bpm/hr and is configurable. In some cases, the rate of increase 264 can be greater than the rate of decrease 254. In some embodiments, pacing rate is increased gradually, over time, to achieve the rate of increase 264, similar to the rate of decrease 254 discussed above. The rate of increase 264 can avoid sudden, non-linear jumps in pacing rate, and can be accomplished by spreading the bpm/hr increase over time. For example, if the rate of increase 264 is 10 bpm/hr, the pacing rate is increased by 1 bpm every 6 minutes until the diurnal pacing rate 266 is accomplished.

For patient comfort or preference, in some embodiments, one or both of the sleep threshold (e.g., when the MTA temperature signal 320 crosses or falls below the XLTA temperature signal 324) and the wake threshold (e.g., when the MTA temperature signal 320 rises above the wake threshold 328) can include an offset to trigger sleep and/or waking to be earlier or later. For example: i) Sleep threshold1 = XLTA - offset1; ii) Sleep threshold2 = XLTA + offset2; iii) Wake Threshold1 = mean(XLTA, NLTA) - offset3; and Wake Threshold2 = mean(XLTA, NLTA) + offset4. In some embodiments, an offset can have a value within a range of approximately -0.25 degrees C to +0.25 degrees C.

In other embodiments, the XLTA temperature signal 324 could be used as both the sleep threshold and wake threshold, and optionally can include an offset to trigger sleep earlier or later and/or waking earlier or later. Nonlimiting examples include: i) Sleep threshold1 = XLTA + offset1; and ii) Wake Threshold1 = XLTA - offset2. In some embodiments, an offset can have a value within a range of approximately -0.25 degrees C to +0.25 degrees C.

In some embodiments, predetermined threshold(s) can be used in place of the XLTA temperature signal 324, the NLTA temperature signal 326, and/or the wake threshold 328. The predetermined threshold(s) can be based, for example, on population samples, temperature data associated with the patient, and the like.

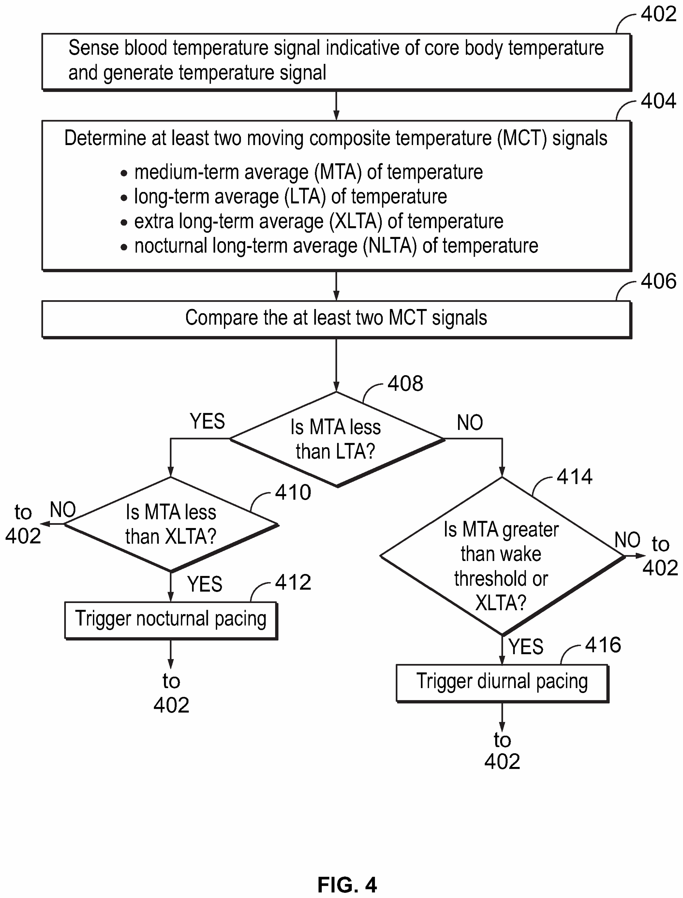

Figure 4 illustrates a process for dynamically adjusting the pacing rate between the diurnal pacing rate 266 and the nocturnal pacing rate 256 in accordance with embodiments herein. In one example, the process of Figure 4 is performed utilizing the temperature signal 316 detected by the temperature sensor 154 and received by the control circuit 156 by the systems and methods described herein. The operations of Figure 4 may be implemented by hardware, firmware, circuitry and/or one or more processors housed partially and/or entirely within the IMD 100, a local external device, remote server or more generally within a health care system. Optionally, the operations of Figure 4 may be partially implemented by an IMD 100 and partially implemented by a local external device, remote server or more generally within a health care system. It should be recognized that while the operations of Figure 4 are described in a somewhat serial manner, one or more of the operations may be continuous and/or performed in parallel with one another. For example, the various operations of the IMD 100 may be continuous and/or performed in parallel with one another and/or other functions of the IMD 100.

For simplicity, the process of Figure 4 will be discussed together with Figures 1B-3. For example, at 402, the one or more processors sense a blood temperature signal indicative of a core body temperature of the patient within which the IMD 100 is implanted. For example, the temperature sensor 154 can sense the blood temperature continuously or at a sampling rate. In some embodiments, one or more processors of the control circuit 156 can sample the blood temperature (e.g., every 5 seconds, more frequently than every 5 seconds) to generate the temperature signal 316.

At 404, the one or more processors determine two or more moving composite temperature (MCT) signals over different periods of time. For example, a first MCT signal is produced based on the temperature signal 316 sensed over a first period of time and a second MCT signal is produced based on the temperature signal 316 sensed over a second period of time that is longer or shorter than the first period of time. In some embodiments, one or more of the MTA temperature signal 320, LTA temperature signal 322, XLTA temperature signal 324, and NLTA temperature signal 326 can be determined. The temperature signals 320, 322, 324, 326 can be determined serially, in parallel, and/or a combination thereof. In other embodiments, one of the MTA temperature signal 320, LTA temperature signal 322, XLTA temperature signal 324, and NLTA temperature signal 326 can be determined, and subsequently compared to predetermined thresholds. Therefore, although Figure 4 is primarily discussed in terms of comparing MCT signals, it should be understood that a single MCT signal, raw signal, other signal acquired by the temperature sensor 154, etc., can be compared to one or more predetermined thresholds to trigger nocturnal pacing and diurnal pacing.

At 406, the one or more processors compare the at least two MCT signals to determine a relationship between the at least two MCT signals. In some embodiments, the one or more processors compare the temperature signal 316 to the first and second MCT signals. In other embodiments, the one or more processors compare more than two MCT signals to determine a relationship between the MCT signals. In still further embodiments, the one or more processors compare the MCT signal(s) to one or more thresholds.

At 408, the one or more processors determine whether the MTA temperature signal 320 is less than the LTA temperature signal 322. In some embodiments a different signal can be compared instead of the MTA temperature signal 320 and the LTA temperature signal 322, such as the temperature signal 316. In still further embodiments, the MTA temperature signal 320 can be compared to a threshold. If yes, flow passes to 410.

At 410, the one or more processors determine whether the MTA temperature signal 320 (or in some embodiments the temperature signal 316) is less than (e.g., falls below) the XLTA temperature signal 324 (or other predetermined threshold). If no, flow returns to 402 to continue to acquire temperature data, and determine and compare signals. If the MTA temperature signal 320 is less than the XLTA temperature signal 324, flow passes to 412 and the one or more processors, such as by using the sleep detection algorithm 252, trigger the rate of decrease 254 to decrease the baseline pacing rate to the nocturnal pacing rate 256.

Returning to 408, if the MTA temperature signal 320 is greater than the LTA temperature signal 322, flow passes to 414 and the one or more processors determine whether the MTA temperature signal 320 (or in some embodiments the temperature signal 316) exceeds or is greater than the waking threshold (e.g., wake threshold 328, XLTA temperature signal 324, predetermined threshold). If no, flow returns to 402 to continue to acquire temperature data, and determine and compare signals. If yes, flow passes to 416 and the one or more processors, such as by using the wake detection algorithm 262, trigger the rate of increase 264 to increase the baseline pacing rate to the diurnal pacing rate 266. In some embodiments, the baseline pacing rate will be the same as the diurnal pacing rate 266 and thus will not be further increased.

Accordingly, the IMD 100 delivers a particular treatment for addressing the biological heart rate differences between wake and sleep states based on core body temperature of a patient. The treatment of the nocturnal pacing rate 256 or the diurnal pacing rate 266 is selected based on a comparison of the current temperature signal (e.g., patient's temperature signal 316, raw temperature signal, MTA temperature signal 320, LTA temperature signal 322) and one or more moving composite signals that are based on the current temperature signal 316 and are determined over different lengths of time (e.g. MTA temperature signal 320, LTA temperature signal 322, XLTA temperature signal 324, NLTA temperature signal 326). In other embodiments, the treatment of the nocturnal pacing rate 256 or the diurnal pacing rate 266 is selected based on a comparison of two or more composite signals that are based on the temperature signal 316 and are determined over different lengths of time.

Further, the IMD 100 delivers the particular treatment which transforms the patient's heart from a state having a pacing rate that supports diurnal or daytime activities to a state having a pacing rate that supports nocturnal needs or sleep, and transforms the patient's heart from a state having a pacing rate that supports nocturnal needs to a state having a pacing rate that supports diurnal needs. As discussed in Figures 2-5, the IMD 100 monitors the core body temperature at a sampling rate to continuously or near continuously, such as in real-time, monitor for a waking state and a sleep state, and thus adjusts the particular treatment as the patient's condition changes in real-time or near real-time.

In some embodiments, the methods and systems herein can be implemented together with other sleep and wake algorithms, such as sleep algorithms that detect sleep based on lack of movement, to confirm the patient's wake and sleep states. Further, the sleep detection and wake detection algorithms discussed herein can be integrated with other monitoring and functionality of the IMD 100, such as arrhythmia detection, shocking, and the like.

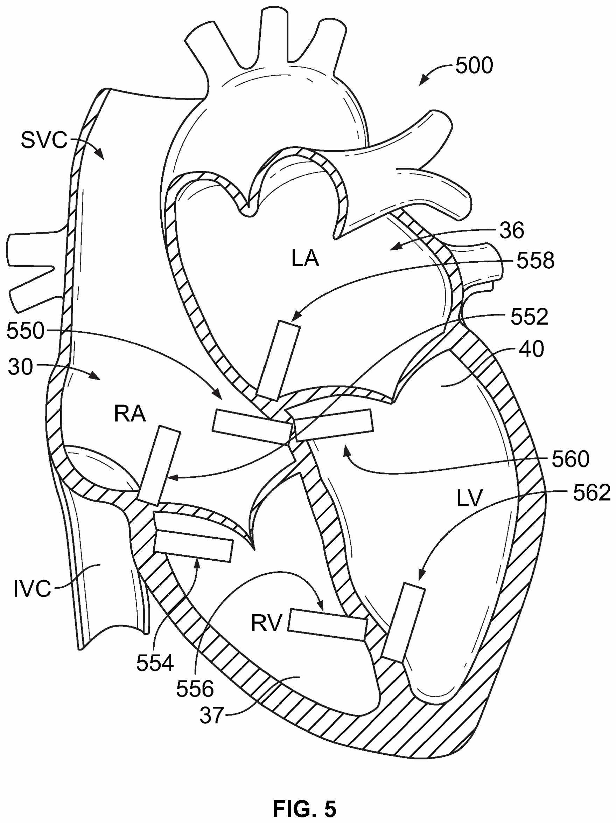

Figure 5 illustrates a plurality of positions 550-562 in which one or more of the IMD 100 can be implanted within a patient's heart 500 in accordance with embodiments herein. It should be understood that there can be other placements of the IMD 100 based on patient structure, structural anomalies, pathology, treatment requirements, and the like. The IMD 100 can be implanted in the right atrium (RA) 30 and is capable of pacing the right ventricle (RV) 37. At 550, the IMD 100 is capable of HISian or para-HISian pacing to produce excitation of the RV 37 and left ventricle (LV) 40. When the IMD 100 is implanted at 552, the IMD 100 is able to provide RA/RV sensing and pacing from the RA 30. When the IMD 100 is implanted at 554, the IMD 100 is able to provide RA/RV sensing and pacing from the RV 37. When the IMD 100 is implanted at 556, the IMD 100 is able to provide RV/LV sensing and pacing from the RV 37. The IMDs 100 implanted at 558, 560, 562 afford left atrium (LA) 36/RA pacing and sensing, LV/RA pacing and sensing, and LV/RV pacing and sensing, respectively. These implementations can produce excitation of the RV 37 and LV 40 in a manner more consistent with normal physiological function.

The temperature measurement could be made by any one or more of the IMDs 100, such as one or more of the IMDs 100 shown at positions 550-562, and the sleep and wake states can be determined by any of the one or more IMDs 100. If more than one IMD 100 is implanted, the more than one IMD 100 can communicate, coordinate, and apply the same system-wide pacing rate. The IMDs 100 can also communicate and coordinate with other IMDs (not shown) to control the pacing rate.

Embodiments may be implemented in connection with the methods and systems described in U.S. Patent Application Number 2023/0405335, entitled "METHODS AND SYSTEMS FOR POSTURE DEPENDENT IMPLANTABLE CARDIOVERTER DEFIBRILLATOR THERAPY"; U.S. Patent 11,273,312, entitled "Method and system for dynamic device-based delay adjustment"; U.S. Patent 7,643,878, entitled "System and method for determining atrioventricular pacing delay based on atrial depolarization"; U.S. Patent 6,832,112, entitled "Method of adjusting an AV and/or PV delay to improve hemodynamics and corresponding implantable stimulation device"; U.S. Patent 7,941,217, entitled "Techniques for promoting biventricular synchrony and stimulation device efficiency using intentional fusion"; U.S. Patent 7,778,706, entitled "Rate adaptive biventricular and cardiac resynchronization therapy"; U.S. Patent 7,702,390, entitled "Rate adaptive biventricular and cardiac resynchronization therapy"; U.S. Patent 11,154,719, entitled "Method and system utilizing a percentage-based atrioventricular delay adjustment"; U.S. Patent 5,741,308, entitled "Dual-chamber implantable pacemaker and method of operating same for automatically setting the pacemaker's AV interval as a function of a natural measured conduction time"; U.S. Patent 8,442,634, entitled "Systems and methods for controlling ventricular pacing in patients with long interatrial conduction delays"; U.S. Patent 11,311,733, entitled "Method and system for adaptive bi-ventricular fusion pacing"; U.S. Patent 10,589,100, entitled "Method and device for pacing latency based multi-point pacing"; U.S. Patent 10,569,091, entitled "Method and device for discrimination of left ventricular pseudo-fusion pacing"; U.S. Patent 10,668,289, entitled "Method and device for controlling rate adaptive pacing based on heart sounds"; U.S. Patent 11,400,297, entitled "Method and device for managing pacing therapy based on interventricular septal activity"; U.S. Patent 10,967,189, entitled "Methods and systems for selectively delivering different types of bi-ventricular pacing"; U.S. Patent 11,311,733, entitled "Method and system for adaptive bi-ventricular fusion pacing"; U.S. Patent 10,173,066, entitled "Methods and systems for selectively delivering different types of bi-ventricular pacing"; U.S. Patent 10,582,874, entitled "Method and device for electrogram based estimation of QRS duration"; U.S. Patent 10,569,091, entitled "Method and device for discrimination of left ventricular pseudo-fusion pacing".

Embodiments may be implemented in connection with one or more implantable medical devices (IMDs). Non-limiting examples of IMDs include one or more of implantable leadless monitoring and/or therapy devices, and/or alternative implantable medical devices. For example, the IMD may represent a cardioverter defibrillator, pacemaker, cardioverter, cardiac rhythm management device, defibrillator, leadless pacemaker and the like. For example, leadless implantable medical device (LIMD) can include one or more structural and/or functional aspects of the device(s) described in U.S. Patent 9,216,285 "Leadless Implantable Medical Device Having Removable And Fixed Components". Additionally or alternatively, the IMD may include one or more structural and/or functional aspects of the device(s) described in U.S. Patent 8,391,980 "Method And System For Identifying A Potential Lead Failure In An Implantable Medical Device" and U.S. Patent 9,232,485 "System And Method For Selectively Communicating With An Implantable Medical Device".

Additionally or alternatively, the IMD may be a subcutaneous IMD that includes one or more structural and/or functional aspects of the device(s) described in U.S. Patent 10,765,860, entitled "Subcutaneous Implantation Medical Device With Multiple Parasternal-Anterior Electrodes"; U.S. Patent 10,722,704, entitled "Implantable Medical Systems And Methods Including Pulse Generators And Leads"; US Patent 11,045,643, entitled "Single Site Implantation Methods For Medical Devices Having Multiple Leads". Further, one or more combinations of IMDs may be utilized from the above recited patents and applications in accordance with embodiments herein.

In some embodiments, the physiologic sensor may be implemented as an accelerometer and may be implemented utilizing all or portions of the structural and/or functional aspects of the methods and systems described in U.S. Patent Application Publication 2021/0345891, entitled "METHOD AND DEVICE FOR DETECTING RESPIRATION ANOMALY FROM LOW FREQUENCY COMPONENT OF ELECTRICAL CARDIAC ACTIVITY SIGNALS"; U.S. Patent Application Publication 2021/0350931, entitled "METHOD AND SYSTEMS FOR HEART CONDITION DETECTION USING AN ACCELEROMETER".

### CLOSING

The various methods as illustrated in the Figures and described herein represent exemplary embodiments of methods. The methods may be implemented in software, hardware, or a combination thereof. In various of the methods, the order of the steps may be changed, and various elements may be added, reordered, combined, omitted, modified, etc. Various of the steps may be performed automatically (e.g., without being directly prompted by user input) and/or programmatically (e.g., according to program instructions).

Various modifications and changes may be made as would be obvious to a person skilled in the art having the benefit of this disclosure. It is intended to embrace all such modifications and changes and, accordingly, the above description is to be regarded in an illustrative rather than a restrictive sense.

Various embodiments of the present disclosure utilize at least one network that would be familiar to those skilled in the art for supporting communications using any of a variety of commercially-available protocols, such as Transmission Control Protocol/Internet Protocol ("TCP/IP"), User Datagram Protocol ("UDP"), protocols operating in various layers of the Open System Interconnection ("OSI") model, File Transfer Protocol ("FTP"), Universal Plug and Play ("UpnP"), Network File System ("NFS"), Common Internet File System ("CIFS") and AppleTalk. The network can be, for example, a local area network, a wide-area network, a virtual private network, the Internet, an intranet, an extranet, a public switched telephone network, an infrared network, a wireless network, a satellite network and any combination thereof.

In embodiments utilizing a web server, the web server can run any of a variety of server or mid-tier applications, including Hypertext Transfer Protocol ("HTTP") servers, FTP servers, Common Gateway Interface ("CGI") servers, data servers, Java servers, Apache servers and business application servers. The server(s) also may be capable of executing programs or scripts in response to requests from user devices, such as by executing one or more web applications that may be implemented as one or more scripts or programs written in any programming language, such as Java^{®}, C, C# or C++, or any scripting language, such as Ruby, PHP, Perl, Python or TCL, as well as combinations thereof. The server(s) may also include database servers, including without limitation those commercially available from Oracle^{®}, Microsoft^{®}, Sybase^{®} and IBM^{®} as well as open-source servers such as MySQL, Postgres, SQLite, MongoDB, and any other server capable of storing, retrieving and accessing structured or unstructured data. Database servers may include table-based servers, document-based servers, unstructured servers, relational servers, non-relational servers or combinations of these and/or other database servers.

The environment can include a variety of data stores and other memory and storage media as discussed above. These can reside in a variety of locations, such as on a storage medium local to (and/or resident in) one or more of the computers or remote from any or all of the computers across the network. In a particular set of embodiments, the information may reside in a storage-area network ("SAN") familiar to those skilled in the art. Similarly, any necessary files for performing the functions attributed to the computers, servers or other network devices may be stored locally and/or remotely, as appropriate. Where a system includes computerized devices, each such device can include hardware elements that may be electrically coupled via a bus, the elements including, for example, at least one central processing unit ("CPU" or "processor"), at least one input device (e.g., a mouse, keyboard, controller, touch screen or keypad) and at least one output device (e.g., a display device, printer or speaker). Such a system may also include one or more storage devices, such as disk drives, optical storage devices and solid-state storage devices such as random access memory ("RAM") or read-only memory ("ROM"), as well as removable media devices, memory cards, flash cards, etc.

Such devices also can include a computer-readable storage media reader, a communications device (e.g., a modem, a network card (wireless or wired), an infrared communication device, etc.) and working memory as described above. The computer-readable storage media reader can be connected with, or configured to receive, a computer-readable storage medium, representing remote, local, fixed and/or removable storage devices as well as storage media for temporarily and/or more permanently containing, storing, transmitting and retrieving computer-readable information. The system and various devices also typically will include a number of software applications, modules, services or other elements located within at least one working memory device, including an operating system and application programs, such as a client application or web browser. It should be appreciated that alternate embodiments may have numerous variations from that described above. For example, customized hardware might also be used and/or particular elements might be implemented in hardware, software (including portable software, such as applets) or both. Further, connection to other computing devices such as network input/output devices may be employed.

Various embodiments may further include receiving, sending, or storing instructions and/or data implemented in accordance with the foregoing description upon a computer-readable medium. Storage media and computer readable media for containing code, or portions of code, can include any appropriate media known or used in the art, including storage media and communication media, such as, but not limited to, volatile and non-volatile, removable and non-removable media implemented in any method or technology for storage and/or transmission of information such as computer readable instructions, data structures, program modules or other data, including RAM, ROM, Electrically Erasable Programmable Read-Only Memory ("EEPROM"), flash memory or other memory technology, Compact Disc Read-Only Memory ("CD-ROM"), digital versatile disk (DVD) or other optical storage, magnetic cassettes, magnetic tape, magnetic disk storage or other magnetic storage devices or any other medium which can be used to store the desired information and which can be accessed by the system device. Based on the disclosure and teachings provided herein, a person of ordinary skill in the art will appreciate other ways and/or methods to implement the various embodiments.

The use of the terms "a" and "an" and "the" and similar referents in the context of describing the disclosed embodiments (especially in the context of the following claims) are to be construed to cover both the singular and the plural, unless otherwise indicated herein or clearly contradicted by context. The terms "comprising," "having," "including" and "containing" are to be construed as open-ended terms (i.e., meaning "including, but not limited to,") unless otherwise noted. The term "connected," when unmodified and referring to physical connections, is to be construed as partly or wholly contained within, attached to or joined together, even if there is something intervening. Recitation of ranges of values herein are merely intended to serve as a shorthand method of referring individually to each separate value falling within the range, unless otherwise indicated herein and each separate value is incorporated into the specification as if it were individually recited herein. The use of the term "set" (e.g., "a set of items") or "subset" unless otherwise noted or contradicted by context, is to be construed as a nonempty collection comprising one or more members. Further, unless otherwise noted or contradicted by context, the term "subset" of a corresponding set does not necessarily denote a proper subset of the corresponding set, but the subset and the corresponding set may be equal.

Operations of processes described herein can be performed in any suitable order unless otherwise indicated herein or otherwise clearly contradicted by context. Processes described herein (or variations and/or combinations thereof) may be performed under the control of one or more computer systems configured with executable instructions and may be implemented as code (e.g., executable instructions, one or more computer programs or one or more applications) executing collectively on one or more processors, by hardware or combinations thereof. The code may be stored on a computer-readable storage medium, for example, in the form of a computer program comprising a plurality of instructions executable by one or more processors. The computer-readable storage medium may be non-transitory.

## Claims

1. An implantable medical device (IMD) (100), comprising:
a temperature sensor (154) configured to sense a temperature signal indicative of a core body temperature of a patient within which the IMD (100) is to be implanted;
one or more pulse generators (116) configured to generate pacing pulses;
one or more electrodes (104, 106) configured to deliver the pacing pulses; and
a control circuit (156) configured to:
produce a first moving composite temperature (MCT) signal based on the temperature signal sensed over a first period of time;
produce a second moving composite temperature (MCT) signal based on the temperature signal over a second period of time, the second period of time being longer than the first period of time;
compare a current temperature signal to the first and second MCT signals; and
control a pacing rate for the pacing pulses based on one or more relations between the current temperature signal, the first MCT signal and the second MCT signal, wherein, to control the pacing rate, the control circuit (156) is configured to change the pacing rate to a nocturnal pacing rate responsive to at least one of:
a) the current temperature signal falls below the second MCT signal; or
b) the first MCT signal falls below the second MCT signal.

2. The IMD of claim 1, wherein, to control the pacing rate, the control circuit (156) is configured to change the pacing rate to a diurnal pacing rate responsive to at least one of:
a) the first MCT signal rises above the second MCT signal; or
b) the first MCT signal rises above a waking threshold.

3. The IMD of any one of claims 1 to 2, wherein, to control the pacing rate, the control circuit (156) is configured to set the pacing rate to a diurnal pacing rate when the current temperature signal exceeds a mathematical combination of the first and second MCT signals.

4. The IMD of any one of claims 1 to 3, wherein, to control the pacing rate, the control circuit (156) is configured to set one or more sleep thresholds based on i) at least one of the current temperature signal, the first MCT signal or the second MCT signal and ii) one or more offsets, the pacing rate changed responsive to at least one of the current temperature signal or the first MCT signal falling below the one or more sleep thresholds.

5. The IMD of claim 4, wherein, to control the pacing rate, the control circuit (156) is configured to set one or more wake thresholds based on i) at least one of the current temperature signal, the first MCT signal or the second MCT signal and ii) one or more offsets, the pacing rate changed responsive to at least one of the current temperature signal or the first MCT signal rising above the one or more wake thresholds.

6. The IMD of claim 4 or 5, wherein the second MCT signal represents a current extra long-term average (XLTA) temperature signal as a trend of daily average temperatures produced by summing a weighted combination of i) a prior XLTA temperature signal and ii) the current temperature signal, the control circuit (156) is configured to set at least one of a sleep threshold or a wake threshold based on the XLTA temperature signal and one or more offsets.

7. The IMD of any one of claims 1 to 6, wherein the first period of time corresponds to one of a) to c) and the second period of time corresponds to another one of a) to c):
a) a duration between 1-15 minutes;
b) a duration between 1-3 hours; and
c) a duration between 1-7 days.

8. The IMD of any one of claims 1 to 7, wherein the second period of time corresponds to a duration of between 1-7 days and the second MCT signal represents a multi-day nocturnal long-term average (NLTA) temperature signal that trends a daily low temperature.

9. The IMD of any one of claims 1 to 8, wherein to control the pacing rate, the control circuit (156) is configured to i) gradually decrease the pacing rate to a nocturnal pacing rate at a rate of decrease, or ii) gradually increase the pacing rate to a diurnal pacing rate at a rate of increase.

## Patentansprüche

1. Implantierbare medizinische Vorrichtung (IMD) (100), umfassend:
einen Temperatursensor (154), der dazu konfiguriert ist, ein Temperatursignal zu erfassen, das eine Körperkerntemperatur eines Patienten angibt, in den die IMD (100) zu implantieren ist;
einen oder mehrere Impulsgeneratoren (116), die dazu konfiguriert sind, Schrittmacherimpulse zu generieren;
eine oder mehrere Elektroden (104, 106), die dazu konfiguriert sind, die Schrittmacherimpulse abzugeben; und
eine Steuerschaltung (156), die zu Folgendem konfiguriert ist:
Erzeugen eines ersten Signals einer veränderlichen kombinierten Temperatur (MCT) basierend auf dem Temperatursignal, das über eine erste Zeitspanne erfasst wird;
Erzeugen eines zweiten Signals einer veränderlichen kombinierten Temperatur (MCT) basierend auf dem Temperatursignal über eine zweite Zeitspanne, wobei die zweite Zeitspanne länger als die erste Zeitspanne ist;
Vergleichen eines aktuellen Temperatursignals mit dem ersten und dem zweiten MCT-Signal; und
Steuern einer Schrittmacherrate für die Schrittmacherimpulse basierend auf einer oder mehreren Beziehungen zwischen dem aktuellen Temperatursignal, dem ersten MCT-Signal und dem zweiten MCT-Signal, wobei, um die Schrittmacherrate zu steuern, die Steuerschaltung (156) dazu konfiguriert ist, die Schrittmacherrate als Reaktion auf mindestens eines der Folgenden zu einer Nacht-Schrittmacherrate zu ändern:
a) das aktuelle Temperatursignal fällt unter das zweite MCT-Signal; oder
b) das erste MCT-Signal fällt unter das zweite MCT-Signal.

2. IMD nach Anspruch 1, wobei, um die Schrittmacherrate zu steuern, die Steuerschaltung (156) dazu konfiguriert ist, die Schrittmacherrate als Reaktion auf mindestens eines der Folgenden zu einer Tag-Schrittmacherrate zu ändern:
a) das erste MCT-Signal steigt über das zweite MCT-Signal; oder
b) das erste MCT-Signal steigt über einen Aufwach-Schwellenwert.

3. IMD nach einem der Ansprüche 1 bis 2, wobei, um die Schrittmacherrate zu steuern, die Steuerschaltung (156) dazu konfiguriert ist, die Schrittmacherrate auf eine Tag-Schrittmacherrate einzustellen, wenn das aktuelle Temperatursignal eine mathematische Kombination aus dem ersten und dem zweiten MCT-Signal überschreitet.

4. IMD nach einem der Ansprüche 1 bis 3, wobei, um die Schrittmacherrate zu steuern, die Steuerschaltung (156) dazu konfiguriert ist, einen oder mehrere Schlaf-Schwellenwerte basierend auf i) mindestens einem aus dem aktuellen Temperatursignal, dem ersten MCT-Signal oder dem zweiten MCT-Signal und ii) einem oder mehreren Versätzen, um die sich die Schrittmacherrate als Reaktion darauf geändert hat, dass mindestens eines aus dem aktuellen Temperatursignal oder dem ersten MCT-Signal unter den einen oder die mehreren Schlaf-Schwellenwerte gefallen ist, einzustellen.

5. IMD nach Anspruch 4, wobei, um die Schrittmacherrate zu steuern, die Steuerschaltung (156) dazu konfiguriert ist, einen oder mehrere Wach-Schwellenwerte basierend auf i) mindestens einem aus dem aktuellen Temperatursignal, dem ersten MCT-Signal oder dem zweiten MCT-Signal und ii) einem oder mehreren Versätzen, um die sich die Schrittmacherrate als Reaktion darauf geändert hat, dass mindestens eines aus dem aktuellen Temperatursignal oder dem ersten MCT-Signal über den einen oder die mehreren Wach-Schwellenwerte gestiegen ist, einzustellen.

6. IMD nach Anspruch 4 oder 5, wobei das zweite MCT-Signal ein aktuelles Extralangzeitmittelwert-Temperatursignal (XLTA-Temperatursignal) als einen Trend für tägliche mittlere Temperaturen, der durch Summieren einer gewichteten Kombination aus i) einem vorherigen XLTA-Temperatursignal und ii) dem aktuellen Temperatursignal erzeugt wird, darstellt und die Steuerschaltung (156) dazu konfiguriert ist, mindestens eines aus einem Schlaf-Schwellenwert oder einem Wach-Schwellenwert basierend auf dem XLTA-Temperatursignal und einem oder mehreren Versätzen einzustellen.

7. IMD nach einem der Ansprüche 1 bis 6, wobei die erste Zeitspanne einem von a) bis c) entspricht und die zweite Zeitspanne einem anderen von a) bis c) entspricht:
a) eine Dauer zwischen 1-15 Minuten;
b) eine Dauer zwischen 1-3 Stunden; und
c) eine Dauer zwischen 1-7 Tagen.

8. IMD nach einem der Ansprüche 1 bis 7, wobei die zweite Zeitspanne einer Dauer zwischen 1-7 Tagen entspricht und das zweite MCT-Signal ein Mehrtages-Nachtlangzeitmittelwert-Temperatursignal (Mehrtages-NLTA-Temperatursignal) darstellt, das zu einer täglichen niedrigen Temperatur tendiert.

9. IMD nach einem der Ansprüche 1 bis 8, wobei, um die Schrittmacherrate zu steuern, die Steuerschaltung (156) dazu konfiguriert ist, i) die Schrittmacherrate schrittweise mit einer Verringerungsrate auf eine Nacht-Schrittmacherrate zu verringern oder ii) die Schrittmacherrate schrittweise mit einer Erhöhungsrate auf eine Tag-Schrittmacherrate zu erhöhen.

## Revendications

1. Dispositif médical implantable (IMD) (100), comprenant :
un capteur de température (154) configuré pour détecter un signal de température indicatif d'une température corporelle centrale d'un patient à l'intérieur duquel le IMD (100) doit être implanté ;
un ou plusieurs générateurs d'impulsions (116) configurés pour générer des impulsions de stimulation ;
une ou plusieurs électrodes (104, 106) configurées pour délivrer les impulsions de stimulation ; et
un circuit de commande (156) configuré pour :
produire un premier signal de température composite mobile (MCT) sur la base du signal de température détecté sur une première période de temps ;
produire un second signal de température composite mobile (MCT) sur la base du signal de température sur une seconde période de temps, la seconde période de temps étant plus longue que la première période de temps ;
comparer un signal de température actuel aux premier et second signaux MCT ; et
commander une fréquence de stimulation pour les impulsions de stimulation sur la base d'une ou plusieurs relations entre le signal de température actuel, le premier signal MCT et le second signal MCT, dans lequel, pour commander la fréquence de stimulation, le circuit de commande (156) est configuré pour modifier la fréquence de stimulation en une fréquence de stimulation nocturne en réponse à au moins l'un parmi :
a) le signal de température actuel tombe en dessous du second signal MCT ; ou
b) le premier signal MCT tombe en dessous du second signal MCT.

2. IMD selon la revendication 1, dans lequel, pour commander la fréquence de stimulation, le circuit de commande (156) est configuré pour modifier la fréquence de stimulation en une fréquence de stimulation diurne en réponse à au moins l'un parmi :
a) le premier signal MCT s'élève au-dessus du second signal MCT ; ou
b) le premier signal MCT s'élève au-dessus d'un seuil d'éveil.

3. IMD selon l'une quelconque des revendications 1 à 2, dans lequel, pour commander la fréquence de stimulation, le circuit de commande (156) est configuré pour régler la fréquence de stimulation à une fréquence de stimulation diurne lorsque le signal de température actuel dépasse une combinaison mathématique des premier et second signaux MCT.

4. IMD selon l'une quelconque des revendications 1 à 3, dans lequel, pour commander la fréquence de stimulation, le circuit de commande (156) est configuré pour régler un ou plusieurs seuils de sommeil sur la base de i) au moins l'un parmi le signal de température actuel, le premier signal MCT ou le second signal MCT et ii) un ou plusieurs décalages, la fréquence de stimulation étant modifiée en réponse au fait qu'au moins l'un parmi le signal de température actuel ou le premier signal MCT tombe en dessous des un ou plusieurs seuils de sommeil.

5. IMD selon la revendication 4, dans lequel, pour commander la fréquence de stimulation, le circuit de commande (156) est configuré pour régler un ou plusieurs seuils d'éveil sur la base de i) au moins l'un parmi le signal de température actuel, le premier signal MCT ou le second signal MCT et ii) un ou plusieurs décalages, la fréquence de stimulation étant modifiée en réponse au fait qu'au moins l'un parmi le signal de température actuel ou le premier signal MCT s'élève au-dessus des un ou plusieurs seuils d'éveil.

6. IMD selon la revendication 4 ou 5, dans lequel le second signal MCT représente un signal de température moyenne à extra long terme (XLTA) actuel en tant que tendance de températures moyennes quotidiennes produite en sommant une combinaison pondérée de i) un signal de température XLTA antérieur et ii) le signal de température actuel, le circuit de commande (156) est configuré pour régler au moins l'un parmi un seuil de sommeil ou un seuil d'éveil sur la base du signal de température XLTA et d'un ou plusieurs décalages.

7. IMD selon l'une quelconque des revendications 1 à 6, dans lequel la première période de temps correspond à l'un parmi a) à c) et la seconde période de temps correspond à un autre de a) à c) :
a) une durée comprise entre 1 et 15 minutes ;
b) une durée comprise entre 1 et 3 heures ; et
c) une durée comprise entre 1 et 7 jours.

8. IMD selon l'une quelconque des revendications 1 à 7, dans lequel la seconde période de temps correspond à une durée comprise entre 1 et 7 jours et le second signal MCT représente un signal de température moyenne à long terme nocturne (NLTA) multidirectionnel qui suit une tendance d'une température basse quotidienne.

9. IMD selon l'une quelconque des revendications 1 à 8, dans lequel pour commander la fréquence de stimulation, le circuit de commande (156) est configuré pour i) diminuer progressivement la fréquence de stimulation jusqu'à une fréquence de stimulation nocturne à une vitesse de diminution, ou ii) augmenter progressivement la fréquence de stimulation jusqu'à une fréquence de stimulation diurne à une vitesse d'augmentation.
